(19) Europäisches Patentamt
European Patent Office
Office européen des brevets

(11) Numéro de publication: **0 605 607 B1**

# (12) FASCICULE DE BREVET EUROPEEN

(45) Date de publication du fascicule du brevet: **29.11.95**

(51) Int. Cl.[6]: **C07C 29/68**, C07C 45/68

(21) Numéro de dépôt: **92920873.4**

(22) Date de dépôt: **21.09.92**

(86) Numéro de dépôt internationale :
**PCT/FR92/00877**

(87) Numéro de publication internationale :
**WO 93/06071 (01.04.93 93/09)**

## (54) PREPARATION D'ENOLATES MANGANEUX ET SES APPLICATIONS.

(30) Priorité: **25.09.91 FR 9111814**

(43) Date de publication de la demande:
**13.07.94 Bulletin 94/28**

(45) Mention de la délivrance du brevet:
**29.11.95 Bulletin 95/48**

(84) Etats contractants désignés:
**AT BE CH DE DK ES FR GB GR IE IT LI LU MC NL SE**

(56) Documents cités:
**FR-A- 2 639 939**

(73) Titulaire: **ELF AQUITAINE**
**Tour Elf,**
**2, Place de la Coupole,**
**La Défense 6**
**F-92400 Courbevoie (FR)**

(72) Inventeur: **CAHIEZ, Gérard**
**68, rue Turbigo**
**F-75003 Paris (FR)**
Inventeur: **CLERY, Patrick**
**3, passage de l'Union**
**F-75007 Paris (FR)**
Inventeur: **LAFFITTE, Jean-Alex**
**26, boulevard des Pyrénées**
**F-64000 Pau (FR)**

(74) Mandataire: **Clisci, Serge**
**S.A. FEDIT-LORIOT & AUTRES**
**CONSEILS EN PROPRIETE INDUSTRIELLE**
**38, Avenue Hoche**
**F-75008 Paris (FR)**

EP 0 605 607 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention a pour objet un nouveau mode de préparation d'énolates manganeux et ses applications.

L'utilité des énolates en général est bien reconnue, surtout depuis les travaux de FLEMING et coll. ("Synthesis" 1976, 736, et "Chem. Soc. Rev." 1981, 10,83); ces composés servent notamment d'intermédiaires dans différentes réactions chimiques. Ils permettent, par exemple, l'obtention de divers esters, cétones ou aldéhydes utiles en parfumerie ou comme matière première pour la production de médicaments ou de pesticides. Des énolates particulièrement intéressants, à savoir énolates manganeux, font l'objet d'une demande de brevet français, publiée sous le n° 2 639 939. Selon ce document, les énolates manganeux sont préparés par l'action de composés organomanganeux mixtes sur des cétones.

Parmi les avantages des énolates du Mn se trouve, par exemple, celui d'éviter la polyalkylation ou polyalkarylation de la cétone, lorsque l'on fait réagir ces énolates avec des halogénures alkyliques ou alkaryliques ; les énolates de métaux alcalins et alcalinoterreux, par contre, en particulier ceux de lithium, conduisent à une polyalkylation ou polyalkarylation plus ou moins poussée. En revanche, certains organomanganeux, en particulier des amidures préparés suivant le procédé rappelé plus haut, donnent des rendements relativement insuffisants dans certaines de leurs applications, tels qu'estérifications ou silylations.

La présente invention apporte un perfectionnement qui, tout en conservant l'avantage de non polyalkylation ou polyalkylarylation, permet d'augmenter sensiblement le rendement des réactions là où les organomanganeux étaient quelque peu en défaut.

Le procédé suivant l'invention,pour l'obtention d'énolates manganeux, est caractérisé en ce qu'un énolate de métal alcalin ou alcalino-terreux est traité par un composé manganeux dans un solvant de l'énolate de Mn à préparer.

Des énolates alcalins et alcalino-terreux, ainsi que leur préparation, étant connus, il n'y a pas lieu de les détailler ici ; on rappellera seulement que ce sont le plus souvent des composés de Na, K, Li, Ca ou Mg, surtout de Li. Leur préparation réside dans l'action d'un organométallique correspondant sur un composé organique porteur d'au moins un carbonyle, notamment sur une cétone. On opère au sein d'un solvant, généralement entre -78°C et +100°C, la température ambiante convenant bien dans beaucoup de cas.

La réaction suivant l'invention, qui constitue une transmétallation, peut être représentée comme suit :

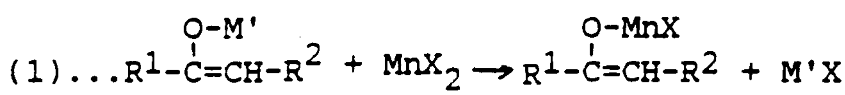

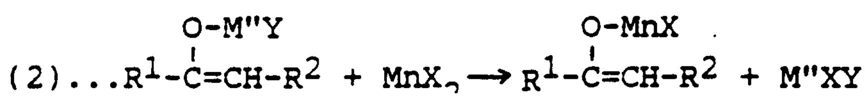

ou

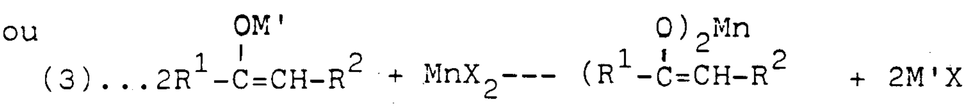

M' étant un métal monovalent, M" divalent, X et Y, semblables ou différents, pouvant être des anions non oxydants ou des groupes aminés. $MnX_2$ peut être remplacé par YMnX. Conviennent bien des halogénures manganeux, notamment $MnCl_2$ pour $MnX_2$.

Les énolates lithiens, c'est-à-dire M' étant Li, conviennent particulièrement bien.

En général, selon la nature des composés mis en jeu, la température du milieu réactionnaire est réglée de façon à respecter la stabilité des réactifs et de manière à obtenir le produit voulu en un laps de temps raisonnable, par exemple en 0,3 à 3 heures. Les températures préférées s'échelonnent entre environ -78 et +100°C et surtout de 10°C à 50°C ; un des avantages du procédé est que la température ambiante convient bien, dans beaucoup de cas, la réaction pouvant être achevée en environ 0,5 à 2 heures à des températures de 15° à 30°C.

Les rapports molaires entre les composés réagissants, suivant les réactions (1) ou (2) susindiquées, sont le plus souvent stoechiométriques, mais il peut être utile d'employer un excès de sel manganeux $MnX_2$ de, par exemple, 0 à 100% par rapport à la stoechiométrie, soit 1 à 2 moles par mole d'énolate, mais en défaut pour réaction (3).

Le procédé de l'invention peut être réalisé avec diverses concentrations des réactifs dans le solvant utilisé ; les concentrations préférées se rangent entre environ 0,1 et 2 moles de composé porteur de groupe carbonyle, par litre de solvant, ou - mieux - entre 0,2 et 0,8 moles.

Parmi les principaux solvants, utilisables, on peut citer des éthers, notamment l'oxyde de diéthyle, le pyrane, le diméthoxy-1,2 éthane et surtout le tétrahydrofurane ; on peut employer, en outre, le diméthylsulfoxyde ou le sulfolane, d'autres solvants étant à la portée de l'homme de l'art. Il est, quelquefois, possible d'améliorer la solubilité du sel manganeux $MnX_2$ dans le solvant par l'adjonction d'un sel alcalin, en particulier d'un sel de Li, suivant la pratique connue dans l'art.

Le procédé de l'invention s'applique à des énolates de métaux alcalins et alcalino-terreux dérivés de nombreuses cétones. Parmi les monocétones correspondantes conviennent les aliphatiques linéaires, telles que

$CH_3(CH_2)_n$-CO-$(CH_2)_mCH_3$

où les nombres n et m, semblables ou différents, sont de 0 à 17. D'autre part, dans une ou chacune de leurs chaînes $(CH_2)_n$ et $(CH_2)_m$, il peut y avoir une double ou triple liaison ou/et un substituant alkyle ou aryle. Des cétones similaires portent à la place d'un ou des deux $CH_3$ terminaux un groupe secondaire ou tertiaire, c'est-à-dire

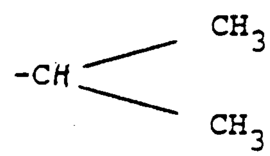

ou

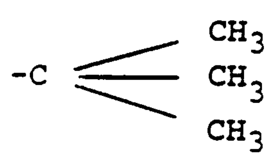

Peuvent également être utilisés des énolates des cétones dans lesquelles l'une ou les deux chaînes $CH_3$-$(CH_2)_n$ ou $CH_3(CH_2)_m$ sont remplacées par un cycle phényle, tolyle, xylyle, naphtyle, cyclopentyle, cyclohexyle ou cyclohexényle pouvant porter un ou plusieurs substituants alkyles. Les cétones de départ peuvent porter des groupes fonctionnels (halogènes, alcoxy, thioalcoxy, etc.).

A titre d'exemples non limitatifs, l'invention peut être réalisée en partant d'énolates correspondant à des cétones comme les diéthyl cétone, dipropyl cétone, di-isopropyl cétone, éthyl propyl cétone, éthyl hexyl cétone, éthyl cyclohexyl cétone, éthyl phényl cétone, butyl-cyclopentanone, méthyl-cyclohexanone, hexyl heptyl cétone, butyl dodécyl cétone, acétophénone, etc.

Ce qui vient d'être dit au sujet des cétones définit la nature des groupes $R^1$ et $R^2$ dans les réactions (1) et (2) illustrées plus haut. Ainsi, alors que $R^2$ peut être H, $R^1$ et $R^2$, semblables ou différents, peuvent être constitués par des chaînes alkyles, alcényles ou alcynyles, en $C_1$ à $C_{20}$ avec préférence pour $C_1$ à $C_{12}$, pouvant comprendre des substituants aryliques ; des cycloalkyles, en particulier cyclopentyle ou cyclohexyle pouvant porter un ou plusieurs substituants d'alkyles en $C_1$ à $C_{12}$ ; conviennent bien des cycles aryles, en particulier phényle, ou naphtyle, portant éventuellement 1 à 3 substituants alkyles en $C_1$ à $C_{12}$ ; ainsi les phényle, tolyle, xylyle, mésityle, mono-, di- ou tri-éthylphényle, dipropyl-phényle etc. forment-ils des énolates intéressants.

Dans le composé manganeux $MnX_2$, X peut être un halogène ou un anion non oxydant d'un composé de S, P, B, C, Si, un groupe oxy- ou thia-hydrocarboné, un groupe amino- etc. Ainsi, par exemple, X est Cl, Br ou I, $CF_3SO_2$,R'COO, $BF_4$, -OR' ou -SR' (R' étant un alkyle ou aryle), -$NR_2$' ou -NR'R'', R', R'' étant des groupes hydrocarbonés, principalement alkyles en $C_1$ à $C_{12}$ ou/et de aryles en $C_6$ à $C_{10}$ .

L'énolate de manganèse formé peut être récupéré du milieu réactionnel par évaporation à l'abri de l'air et de l'humidité du solvant, ou par tout autre moyen connu, pour être ensuite transformé en le dérivé voulu. Cependant, dans la plupart des applications courantes, il est aisé de traiter cet énolate in situ, dans son solvant d'origine, éventuellement en ajoutant un autre solvant ou/et modifiant la température, avant d'introduire un nouveau réactif adéquat. C'est ainsi que l'on peut acyler, silyler, alkyler, halogéner, hydroxyalkyler, etc. le produit dans son milieu réactionnel par l'addition d'un anhydride ou d'un chlorure d'acide,d'un halogénure de trialkylsilyle, d'halogénure d'allyle ou d'alkyle, d'un halogène, d'un aldéhyde, puis de l'eau, etc. Le manganèse s'éliminant au cours de telles réactions, on aboutit aux esters d'énols concernés,ou à des $\beta$-dicétones, aux dérivés silylés,ou aux cétones correspondantes, alkylées, allylées ou halogénées, etc. On peut de même aldoliser la cétone de départ.

EXEMPLE 1

Préparation d'un énolate manganeux correspondant à la dipropyl cétone et son application à la benzylation en $\alpha$ de celle-ci.

La matière de départ est l'énolate de lithium correspondant,

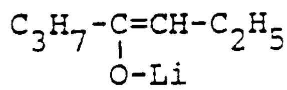

obtenu par la réaction du di-isopropyl amidure de Li, soit

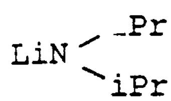

avec la dipropyl cétone

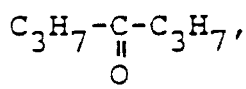

à la température ambiante.

A. - A 100 mmoles d'énolate de Li, dissous dans 150 ml de tétrahydrofurane (THF), on ajoute 125 mmoles de $MnCl_2$ pris sous la forme de son sel double $MnCl_2.2LiCl$ dans 200 ml de THF. Le mélange est agité durant 1 heure à 20 ° C, ce qui conduit à la transmétallation :

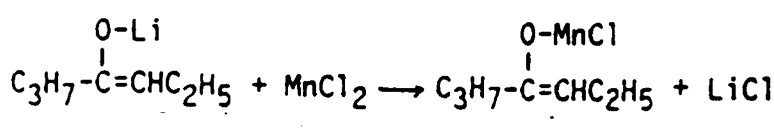

A la solution obtenue on ajoute 125 mmoles de (goutte à goutte) bromure de benzyle $PhCH_2Br$ et 75 ml de diméthyl sulfoxyde (DMSO). Après 1 heure d'agitation à 20 ° C on constate la formation de dipropyl cétone benzylée en $\alpha$ de la fonction cétone, avec un rendement de 94% par rapport à l'heptanone de départ.

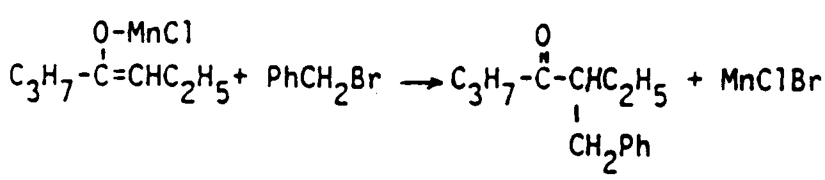

dipropyl cétone ($E_b^{0,01}$ = 85 ° C) $\alpha$ - benzylée Aucune polybenzylation n'a lieu.

B. - En partant du même énolate de Li qu'en A, au lieu d'effectuer une transmétallation, on fait tout de suite agir l'halogénure de benzyle, en agitant 100 mmoles de l'énolate de Li, dans 150 ml de THF, avec 125 mmoles de $PhCH_2Br$ et 75 ml de DMSO, pendant 1 heure à 20 ° C. On a alors :

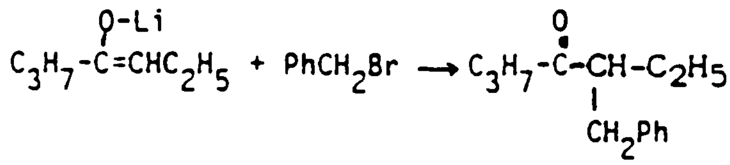

(benzyl-3 heptanone-4) mais la cétone benzylée ainsi n'est obtenue qu'avec un rendement de 68% (contre 94% en A), et elle est accompagnée de produits polybenzylés formés à raison de 23%, ces produits étant du type :

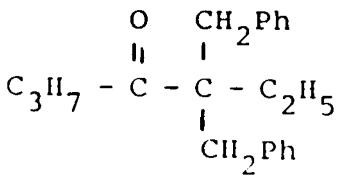

ou

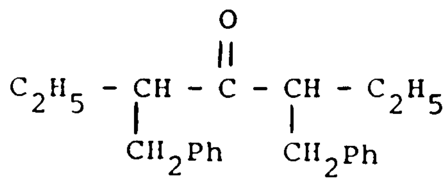

Ainsi voit-on que la transformation de l'énolate de Li en celui de Mn a apporté des avantages marqués.

EXEMPLE 2

Préparation d'un énolate manganeux et sa silylation.

A. - Un essai a consisté à employer, au départ, un énolate de Mn non pas préparé directement par l'action d'un organo-Mn sur la cyclohexanone, mais par transmétallation à partir de l'énolate lithié, de manière similaire à A de l'exemple 1,

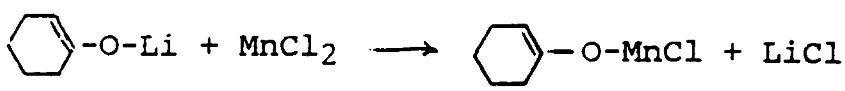

Alors, la silylation, effectuée comme sous B (voir plus loin), est faite avec un rendement de 77%.

B. - Dans un autre essai, on prépare un énolate par l'action de 100 mmoles de N-méthyl phényl amidure de phénylmanganèse,

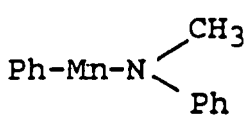
,

sur 100 mmoles de cyclohexanone, dans 200 ml de THF, a 20 °C pendant 1 heure :

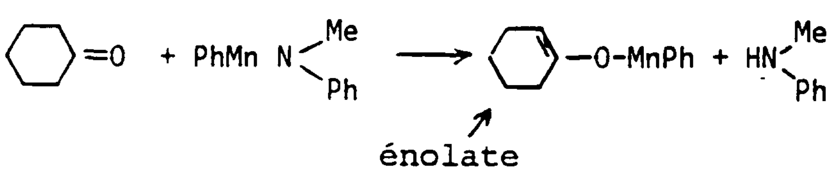


A la solution obtenue on ajoute 100 mmoles de triméthyl chlorosilane $ClSiMe_3$ à 20 °C, et l'on agite durant 30 minutes pour produire la réaction :

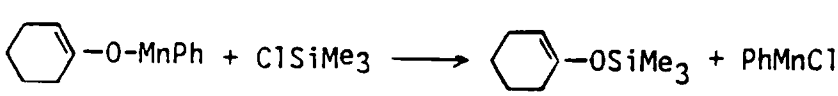

La 1-triméthylsilyloxycyclohexènone est obtenue avec un rendement de 59% contre 77% pour le mode opératoire A.

EXEMPLE 3

Des préparations analogues à celles de l'exemple 2 sont faites en partant de la 3-méthyl cyclohexanone à la place de la cyclohexanone. On procède de même en B à partir de

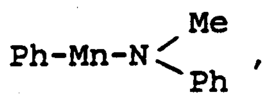

et, par contre, en A par transmétallation de l énolate lithien en énolate manganeux. La silylation s'opère alors avec les rendements de :

79 % pour A

et 67 % pour B.

EXEMPLE 4

Des essais similaires à ceux des exemples 2 et 3 sont effectués en partant de la nonanone

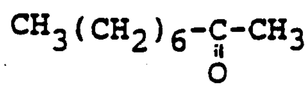

Les rendements en composé silylé

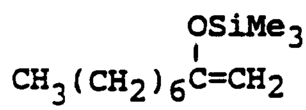

sont dans ce cas, pour A - 78% pour B - traces.

Il apparaît que l'avantage de la transmétallation, en vue de l'obtention de l'énolate manganeux, est particulièrement marqué pour les produits dérivés des cétones possédant un méthyle en $\alpha$ du carbonyle.

EXEMPLES 5 à 7

La technique de l'exemple 1, A et B, a été appliquée à la préparation d'énolate de Mn à partir de la cyclohexanone, de 2-méthylcyclohexanone et de la 3-méthylcyclohexanone. Chacun de ces énolates de Mn fut ensuite soumis à l'alkylation au moyen de bromure de benzyle $C_6H_5CH_2Br$, comparativement avec la même alkylation appliquée à de l'énolate de lithium correspondant. Le tableau ci-après donne les rendements % de l'obtention de la cétone benzylée, ainsi que le % de polybenzylation.

| Ex. | Mode | Cétone de départ | Enolate de : | Rdt % | % ce polyalkylation |
|---|---|---|---|---|---|
| 5 | A | Cyclohexanone | Mn par transmétallation | 88 | 0 |
| 5 | B | " | Li | 55 | 32 |
| 6 | A | 2-méthyl" | Mn par transmet. | 85 | 0 |
| 6 | B | " " " | Li | 65 | 25 |
| 7 | A | 3-méthyl" | Mn par transmet. | 79 | 0 |
| 7 | B | " " " | Li | 37 | 47 |

Rapprochés de ceux de l'exemple 1, ces résultats montrent que le rendement est amélioré par l'invention tant pour les cétones de départ linéaires que pour les cétones cycliques, et il en est de même en ce qui concerne la polyalkylation. Celle-ci est complètement supprimée dans le procédé suivant l'invention, alors qu'elle prend des proportions sensibles dans l'art antérieur, comme d'ailleurs signalé par H. HOUSE dans Organic Syntheses Cell. Vol. VI, 1988, 121 et par H. HOUSE et al dans J. Org. Chem. 1971, 36, 2361.

EXEMPLE 8

Application à l'aldolisation d'une cétone.

Par transmétallation de l'énolate lithien dérivé de la pentanone on a préparé l'énolate manganeux, de manière analogue à celle de l'exemple 1-A :

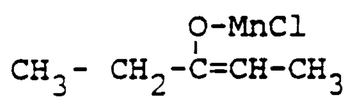

A 200 ml de THF contenant 100 mmoles de ce composé, on a mélangé 100 mmoles de benzaldéhyde, $C_6H_5CHO$, en 1 minute à -78 °C. Ensuite, par l'action de l'eau, on a obtenu :

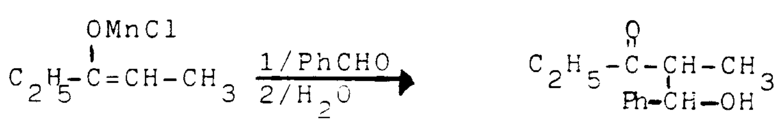


EXEMPLE 9

Application de l'invention à l'aldolisation d'une cétone.

A l'énolate manganeux de la diéthyl cétone, préparé selon le mode opératoire décrit ci-dessus, sont ajoutées, à -78 °C, 50 mmoles (5,1 ml) de benzaldéhyde. Après 10 secondes d'agitation à -78 °C, le milieu réactionnel est hydrolysé par addition, goutte à goutte, d'une solution saturée de $(NH_4)_2SO_4$. Après décantation, filtration sur hyflo-supercel et évaporation des solvants sous vide, la 5-phényl-5-hydroxy 4-méthyl pentan 3-one est isolée par chromatographie liquide à basse pression. Gel de silice 15 µm. Eluant : cyclohexane/acétate d'éthyle = 90/10. Rdt = 72 %. Erythro/thréo = 61/39.

EXEMPLE 10

Préparation d'un énolate manganeux d'ester.

A 50 mmoles (5,05 g) de diisopropyl amine en solution dans 60 ml de THF, sont ajoutées, à -30 ° C, 50 mmoles de BuLi / hexane. Le milieu réactionnel est maintenu sous agitation à la température ambiante pendant 30 minutes puis 40 mmoles (4,08 g) de propionate d'éthyle sont additionnées, goutte à goutte, en 15 minutes à -78 ° C. L'agitation est maintenue pendant 15 minutes à cette température puis 50 mmoles (10,55 g) du complexe $MnCl_4Li_2$, en solution dans 80 ml de THF, sont ajoutées, goutte à goutte, en 10 minutes. Le milieu réactionnel est maintenu sous agitation à la température ambiante pendant 30 minutes (coloration orangée).

EXEMPLE 11

Aldolisation d'un ester.

A l'énolate manganeux du propionate d'éthyle, préparé comme indiqué ci-dessus, sont ajoutées, à -78 ° C, 50 mmoles (5,10 ml) de benzaldéhyde. Après 10 secondes d'agitation à -78 ° C, le milieu réactionnel est hydrolysé par addition, goutte à goutte, d'une solution saturée de $(NH_4)_2SO_4$. Après décantation, filtration sur hyflo-supercel et évaporation des solvants sous vide, le 3-phényl 3-hydroxy 2-méthyl propionate d'éthyle est isolé par chromatographie liquide à basse pression. Gel de silice 15 $\mu$m. Eluant : cyclohexane/acétate d'éthyle = 90/10. Rdt = 72 % . Erythro/thréo = 61/39.

**Revendications**

1. Procédé de préparation d'énolates manganeux, caractérisé en ce que des énolates de métaux alcalins ou alcalino-terreux sont traités par des composés manganeux au sein d'un solvant de l'énolate de Mn à préparer.

2. Procédé suivant la revendication 1, caractérisé en ce que le traitement, qui constitue une transmétallisation, est effectué au moyen d'un énolate de Na, K, Li, Ca ou Mg, et plus particulièrement à l'aide d'un énolate de lithium.

3. Procédé suivant la revendication 1 ou 2, caractérisé en ce que le composé manganeux est du type $MnX_2$ ou YMnX , X et Y étant des anions non oxydants ou groupes aminés, semblables ou différents.

4. Procédé suivant une des revendications précédentes, caractérisé en ce que le traitement est effectué entre -78 ° C et + 100 ° C; en particulier entre 10 ° et 50 ° C, en 0,3 à 3 heures ou, de préférence, durant 0,5 à 2 heures.

5. Procédé suivant une des revendications précédentes, caractérisé en ce que le rapport molaire, composé manganeux/énolate est de 0,5 à 2.

6. Procédé suivant une des revendications précédentes, caractérisé en ce que chacun des réactifs se trouve dans le solvant à une concentration de 0,1 à 2 moles par litre de solvant.

7. Procédé suivant une des revendications précédentes, caractérisé en ce que l'énolate soumis à la transmétallation est

(1) 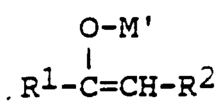 ou (2) 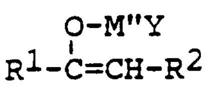

M' étant un métal monovalent, M'' divalent, Y un anion non oxydant ou un groupe aminé, $R^1$ et $R^2$, semblables ou différents étant des : alkyles, alcényles ou alcynyles en $C_1$ à $C_{20}$, avec préférence pour $C_1$ à $C_{12}$ , pouvant porter des substituants aryliques ; des cycloalkyles, particulièrement en $C_5$ ou $C_6$ , pouvant porter des substituants alkyles en $C_1$ à $C_{12}$; ou/et des aryles en $C_6$ à $C_{10}$; $R^2$ pouvant

également être un atome d'hydrogène.

**8.** Procédé suivant la revendication 7, dans lequel M' est le lithium.

**9.** Application du procédé suivant une des revendications précédentes à l'obtention d'un énol acylé, silylé, alkylé, halogéné ou hydroxyalkylé.

**10.** Application du procédé suivant une des revendications 1 à 8 à l'obtention d'une cétone acylée, alkylée, halogénée, allylée ou hydroxyalkylée.

**11.** Application suivant la revendication 10, caractérisée en ce que l'on fait réagir d'abord une cétone avec un dérivé organique du lithium, au sein d'un solvant, pour former un énolate de Li, que l'on agite ensuite la solution avec un sel de manganèse, et que la solution d'énolate de Mn, ainsi obtenue, est mélangée avec un halogénure d'alkyle, d'alkaryle ou d'aryle, de façon à fixer un seul alkyle, alkaryle ou aryle sur la cétone de départ.

**12.** Nouveau produit chimique, constitué par un di-énolate manganeux du type

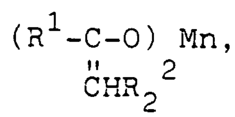

où $R^1$ et $R^2$ ont la même signification que dans la revendication 7.

**Claims**

**1.** A method for preparing manganous enolates, which comprises treating alkali metal or alkaline earth metal enolates with manganous compounds in a solvent for the Mn enolate to be prepared.

**2.** A method according to claim 1 wherein the treatment, which constitutes a transmetalation, is effected by means of an Na, K, Li, Ca or Mg enolate and more particularly with a lithium enolate.

**3.** A method according to claim 1 or 2 wherein the manganous compound is of the type $MnX_2$ or YMnX, X and Y being identical or different non-oxidizing anions or amino groups.

**4.** A method according to any one of the preceding claims wherein the treatment is effected at a temperature between -78°C and +100°C, in particular between 10° and 50°C, for a duration of from 0.3 to 3 hours or, preferably, for a duration of from 0.5 to 2 hours.

**5.** A method according to any one of the preceding claims wherein the molar ratio manganous compound/enolate is from 0.5 to 2.

**6.** A method according to any one of the preceding claims wherein each of the reactants is present in the solvent at a concentration of from 0.1 to 2 mol per liter of solvent.

**7.** A method according to any one of the preceding claims wherein the enolate subjected to transmetalation is

(1) 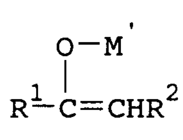 or (2) 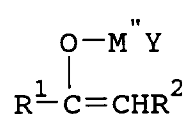

M' being a monovalent metal, M" being a divalent metal, Y being a non-oxidizing anion or an amino group and $R^1$ and $R^2$, which are identical or different, being each an alkyl, alkenyl or alkynyl group having from 1 to 20 carbon atoms and preferably from 1 to 12 carbon atoms which can carry aryl

substituents, or a cycloalkyl group in particular a ($C_5$-$C_6$)-cycloalkyl group, which can carry ($C_1$-$C_{12}$)-alkyl substituents ; and/or a ($C_6$-$C_{10}$)-aryl group, it also being possible for $R^2$ to be a hydrogen atom.

**8.** A method according to claim 7 in which M' is lithium.

**9.** The use of the method according to any one of the preceding claims for preparing an acylated, silylated, alkylated, halogenated or hydroxyalkylated enol.

**10.** The use of the method according to any one of claims 1 to 8 for preparing an acylated, alkylated, halogenated, allylated or hydroxyalkylated ketone.

**11.** The use according to claim 10 wherein firstly a ketone is reacted with an organolithium derivative in a solvent to form an Li enolate, the solution is then stirred with a manganese salt and the resulting solution of Mn enolate is mixed with an alkyl, alkaryl or aryl halide so as to attach a single alkyl, alkaryl or aryl to the starting ketone.

**12.** A novel chemical product consisting of a manganous dienolate of the type

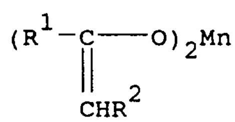

in which $R^1$ and $R^2$ are as defined in claim 7.

**Patentansprüche**

**1.** Verfahren zur Herstellung von Mangan(II)-enolaten, dadurch gekennzeichnet, daß Enolate von alkalischen oder erdalkalischen Metallen durch Mangan(II)-Verbindungen in einem Lösungsmittel des herzustellenden Mn-Enolats behandelt werden.

**2.** Verfahren nach Anspruch 1, dadurch gekennzeichnet, daß die Behandlung, die eine Transmetallisation darstellt, mit Hilfe eines Na-, K-, Li-, Ca- oder Mg-Enolats und insbesondere mit Hilfe eines Lithiumenolats durchgeführt wird.

**3.** Verfahren nach Anspruch 1 oder 2, dadurch gekennzeichnet, daß die Mangan(II)-Verbindung vom Typ $MnX_2$ oder YMnX ist, wobei X und Y nichtoxidierende Anionen oder Aminogruppen und gleich oder verschieden sind.

**4.** Verfahren nach einem der vorhergehenden Ansprüche, dadurch gekennzeichnet, daß die Behandlung zwischen -78°C und +100°C, insbesondere zwischen 10° und 50°C, in 0,3 bis 3 Stunden oder vorzugsweise während 0,5 bis 2 Stunden durchgeführt wird.

**5.** Verfahren nach einem der vorhergehenden Ansprüche, dadurch gekennzeichnet, daß das Molverhältnis Mangan(II)-Verbindung/Enolat 0,5 bis 2 beträgt.

**6.** Verfahren nach einem der vorhergehenden Ansprüche, dadurch gekennzeichnet, daß sich jedes der Reagenzien in dem Lösungsmittel in einer Konzentration von 0,1 bis 2 Mol pro Liter Lösungsmittel befindet.

**7.** Verfahren nach einem der vorhergehenden Ansprüche, dadurch gekennzeichnet, daß das der Transmetallisation unterzogene Enolat

(1) 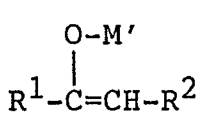 oder (2) 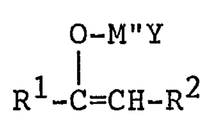

ist, wobei M' ein einwertiges Metall, M'' zweiwertig, Y ein nicht oxidierendes Anion oder eine Aminogruppe ist, $R^1$ und $R^2$, die gleich oder verschieden sind, sind: Alkyle, Alkenyle oder Alkynyle zu $C_1$ bis $C_{20}$, vorzugsweise $C_1$ bis $C_{12}$, die Alkylsubstituenten tragen können; Cycloalkyle insbesondere zu $C_5$ oder $C_6$, die Alkylsubstituenten zu $C_1$ bis $C_{12}$ tragen können; oder/und Aryle zu $C_6$ bis $C_{10}$; wobei $R^2$ auch ein Wasserstoffatom sein kann.

**8.** Verfahren nach Anspruch 7, in welchem M' Lithium ist.

**9.** Anwendung des Verfahrens nach einem der vorhergehenden Ansprüche auf die Herstellung eines azylilerten, silylierten, alkylierten, halogenierten oder hydroxyalkylierten Enols.

**10.** Anwendung des Verfahrens nach einem der Ansprüche 1 bis 8 auf die Herstellung eines azylierten, alkylierten, halogenierten, allylierten oder hydroxyalkylierten Ketons.

**11.** Anwendung nach Anspruch 10, dadurch gekennzeichnet, daß man zunächst ein Keton mit einem organischen Derivat von Lithium in einem Lösungsmittel reagieren läßt, um ein Li-Enolat zu bilden, daß man anschließend die Lösung mit einem Mangansalz rührt und daß die so erhaltene Mn-Enolat-Lösung mit einem Alkyl-, Alkaryl- oder Arylhalogenid gemischt wird, so daß ein einziges Alkyl, Alkaryl oder Aryl an dem Ausgangsketon fixiert wird.

**12.** Neues chemisches Produkt, bestehend aus einem Mangan(II)-dienolat vom Typ

$$(R^1\text{-}\underset{\underset{CHR_2}{\|}}{C}\text{-}O)_2\,Mn,$$

in welchem $R^1$ und $R^2$ dieselbe Bedeutung wie in Anspruch 7 haben.